# EUROPÄISCHE PATENTSCHRIFT

(11) **EP 4 175 703 B1**
(45) Veröffentlichungstag und Bekanntmachung des Hinweises auf die Patenterteilung: **02.10.2024**
(21) Anmeldenummer: 21722237.1
(22) Anmeldetag: 03.05.2021
(51) Int. Cl.: A63B 21/00, A63B 23/18, A61M 16/00, A61M 16/20, A61M 16/08, A63B 71/06

(54) **ATEMWEGSTHERAPIEGERÄT**
RESPIRATORY THERAPY APPARATUS
APPAREIL DE THÉRAPIE RESPIRATOIRE

(30) Priorität: 11.08.2020 EP 20190395
(43) Veröffentlichungstag der Anmeldung: 10.05.2023
(73) Patentinhaber: CEGLA Medizintechnik GmbH, 56410 Montabaur (DE)
(72) Erfinder: EBINGER, Andrea, 56410 Montabaur (DE)
(74) Vertreter: Christ, Niko
(86) Internationale Anmeldenummer: PCT/EP2021/061531
(87) Internationale Veröffentlichungsnummer: WO 2022/033734

(56) Entgegenhaltungen:
- EP-A1- 0 262 239
- EP-A1- 3 620 195
- EP-B1- 0 262 239
- WO-A1-2019/070804
- DE-T2- 69 305 677
- US-A1- 2019 201 743
- US-B1- 10 004 872

## Beschreibung

Die Erfindung bezieht sich auf ein Atemwegstherapiegerät zur Behandlung der Muskulatur von Atemwegen, Lungenfunktionen und/oder zur Schleimlösung im Lungen-, Nasen- und/oder Rachenraum eines Patienten gemäß des Patentanspruchs 1.

Ein solches Atemwegstherapiegerät ist beispielsweise in EP 3 251 718 B1 beschrieben. In einem Rohrstück ist dort ein biegeelastischer Körper in Form eines Schlauches an einem Mundstück befestigt. Das Rohrstück ist gekrümmt, sodass der Schlauch eine Krümmung und folglich eine Querschnittsverringerung im Krümmungsbereich aufweist. Sobald ein Patient durch das Mundstück Atemluft einsaugt oder auspresst, wird die Umgebungsluft bzw. die Atemluft durch den Schlauch gezogen bzw. gedrückt, sodass dieser in eine Schwingung oder Vibration gelangt. Durch diese Vibration entstehen oszillierende Luftdruckschwankungen im Mund-, Nasenraum und/oder der Lunge des Patienten, wodurch sowohl die Muskulatur der Atemwege als auch die Schleimlösung in diesen Bereichen entsteht.

Durch die EP 3 620 195 A1 ist eine Beatmungs-Vorrichtung bekannt geworden, die dazu dient, einen künstlich beatmeten Patienten zu versorgen und gleichzeitig die Verschleimung der Bronchien zu lösen und gleichzeitig die Verschleimung der Bronchien zu lösen sowie die Atemmuskulatur zu trainieren. Diese Vorrichtung besteht aus einem Atemwegstherapiegerät, beispielsweise der eingangs zitierten Gattung.

Solche Atemwegstherapiegeräte haben sich in der Praxis seit Jahrzehnten bewährt und werden zur Behandlung von Patienten erfolgreich verwendet. Gleichwohl hat sich nachteiliger Weise herausgestellt, dass der verwendete Schlauch zum einen eine sehr geringe Lebensdauer aufweist und zum anderen bereits nach wenigen Atemzügen verstopfen kann. Darüber hinaus werden im Inneren des Schlauches Aerosolen, Schleim oder sonstige Bakterien bzw. Viren abgelagert, die nach jeder Behandlung zu entfernen sind. Somit ist der Schlauch nach wenigen Behandlungsvorgängen aufwendig aus dem Rohrstück und dem Mundstück zu entnehmen und beispielsweise in einer Mikrowelle oder in einem sonstigen zur Sterilisation geeigneten medizinischen Gerät zu verbringen, um eine solche Keimlösung zu erreichen.

Wie jede Muskulatur kann auch die Atemmuskulatur trainiert werden. Beim Muskeltraining unterscheiden die Mediziner zwischen einem Stärketraining und einem Ausdauertraining. Die bisherigen Geräte zum Training der Atemmuskulatur sind entweder resistive (Widerstand erzeugende Geräte) oder sogenannte Thresholdgeräte. Bei Thresholdgeräten öffnet sich bei Überwindung eines gewissen Druckes ein Federventil und gibt eine Öffnung frei.

Werden resistive Atemmuskeltrainer verwendet, dann wird in der Regel der Atmung eine Stenose vorgeschaltet.

Zum Training der Muskulatur muss ein gewisser Widerstand überwunden werden. Wenn ein vergleichbares Training durchgeführt werden soll, muss dieser Widerstand bei jedem Training verständlicherweise identisch sein.

Alle bisher auf den Markt gekommenen Geräte sind weitgehend für die Ein- oder Ausatmung bestimmt und verfügen ausschließlich nur über statische Druckerzeugung (PIP =POSITIVE INSPIRATORY PRESSURE).

Ein Atemtrainingsgerät, das inspiratorisch oszillierend, resistive (OPIP) und thresholdgetrieben die Bronchien erweitert und darin festsitzenden Schleim löst oder verflüssigt, gibt es im Zusammenhang mit dem Atemmuskeltraining bisher somit nicht.

Weitere solche Atemwegstherapiegeräte sind beispielsweise der US 10,004,872 B1, EP 0 262 239, US 2019/0201743 A1 oder WO 2019/070804 A1 zu entnehmen. Den dort beschriebenen Atemwegstherapiegeräten ist gemeinsam, dass in einem Strömungskanal ein Ventil in Form einer aufschwingenden Platte oder Zunge angeordnet ist, durch den in Abhängigkeit von den erzeugten Luftdrücken, also des Einatmungs- oder Ausatmungsdruckes, der von einem Patienten generiert ist, Druckschwankungen entstehen bzw. gebildet sind. Dabei strömt die eingesaugte oder ausgepresste Luft senkrecht auf eine Fläche der Zunge oder Platte, die von dem vorherrschenden Druck der Luft von der Halteplatte abgehoben ist, sodass die Luft durch den Luftspalt zwischen der Zunge und der Oberseite der Halteplatte strömen kann.

Bei solchen Atemwegstherapiegeräten ist als nachteilig anzusehen, dass die eingesaugte oder ausgeatmete Luft senkrecht auf die Zunge einwirkt, denn dadurch entsteht keine Vibration der Zunge. Diese kann nämlich lediglich abgehoben oder geschlossen sein. Dies entspricht einer bekannten Ventilfunktion Eine Schwingung der Zunge entsteht durch diese Anordnung und der damit verbundenen Luftströmung nicht.

Darüber hinaus lässt sich bei den bekannt gewordenen Atemwegstherapiegeräten die Luftmenge, die in den Durchlasskanal des jeweiligen Hohlköpers eingesaugt oder ausgepresst ist, nicht individuell patientengerecht einstellen. Vielmehr gelangt eine konstante vorgegebenen Luftmenge in den Durchlasskanal des Hohlkörpers bzw. wird aus diesem ausgepresst. Dieser Volumenanteil der eingesaugten oder ausgepressten Luft entspricht dabei dem Lungenvolumen des jeweiligen Patienten. Da jedoch eine Vielzahl von unterschiedlichen Patienten und deren Erkrankungen oder Trainingszwecken mit Hilfe eines Atemwegstherapiegerätes zu behandeln sind, ist die Einstellmöglichkeit bei den bekannt gewordenen Atemwegstherapiegeräten sehr begrenzt.

Der Erfindung liegt daher die Aufgabe zugrunde, ein Atemwegstherapiegerät der eingangs genannten Gattung derart weiterzubilden, dass eine oszillierende, resistive und/oder threshold typische Luftdruckschwingungen entstehen lässt, bei dem die individuellen Widerstände bei der Ein- und Ausatmung individuell Patienten gerecht, einstellbar ist und eine Umrüstung oder Umstellung des Atemwegstherapiegerätes nicht erforderlich ist. Je nach Länge, Schwere, Steifigkeit und Lage der Zunge sowie der Ausgestaltung oder der Anordnung des Hohlraums kann die Trainingslast zusätzlich individuell angepasst werden.

Diese Aufgabe ist durch die Merkmale des Patentanspruchs 1 gelöst.

Weitere vorteilhafte Weiterbildungen der Erfindung ergeben sich aus den Unteransprüchen.

Dadurch, dass in den Hohlkörper ein Haltekörper eingesetzt ist, in den mindestens eine Öffnung eingearbeitet ist, dass an dem Haltekörper im Bereich der Öffnung eine frei schwingende und aus einem metallischen Werkstoff hergestellte Zunge befestigt ist, durch die die Öffnung des Haltekörpers in Abhängigkeit von den im Inneren des Hohlkörpers vorherrschenden Luftströmungen verschlossen oder bereichsweise freigegeben ist, dass die dem Mundstück gegenüberliegende Stirnseite des Hohlkörpers offen ist, dass in diese Stirnseite eine Stenose eingesetzt ist, in deren Mantelfläche mindestens zwei Durchgangsöffnungen vorgesehen sind, durch die Umgebungsluft in das Innere des Hohlkörpers strömen oder vice versa austreten, dass die Durchgangsöffnungen der Stenose unterschiedliche Querschnittsflächen als Luftdurchlässe für die Luftströmungen aufweisen, und dass die Stenose in dem Hohlkörper drehbar gelagert ist, derart dass durch Verdrehen der Stenose eine der Durchgangsöffnungen als Luftdurchlass auswählbar bzw. einstellbar ist, soll eine auf jeden Patienten individuell einstellbare Regulierung des Druckzustandes im Inneren des Hohlkörpers erreicht sein. Durch diesen Mechanismus werden somit oszillierende Druckschwankungen verschiedenster Form erzeugt, die zur Bronchialerweiterung, stabilisierend und Schleim lösend wirksam sind. Ein solches Atemwegstherapiegerät ist folglich für individuelle medizinische Atemmuskulatur-Trainingszwecke einsetzbar, bei dem beim Einsaugen und/oder Ausatmen der Atemluft sowohl eine oszillierende, eine resistive oder threshold getriebene Bronchienerweiterung zur Verfügung gestellt ist.

Der Erfindungsgegenstand verwendet Zungen zur Erzeugung von akustisch wahrnehmbaren Oszillationen, die durch ihre unterschiedliche Frequenz die Inspiration von der Exspiration unterscheiden lassen. Je nach Beschaffenheit und Lage wird ein unterschiedlich hoher, definierter Druck bzw. Fluss benötigt, um die Zungen zum Schwingen zu bringen. Durch Vorschalten unterschiedlicher Stenosen kann der Druck bzw. Fluss, bei dem die Zunge zu schwingen beginnt, variiert und eingestellt werden. Wird der gewünschte Druck bzw. Fluss erreicht, entsteht ein hörbarer Ton. Bei Nutzung des Gerätes als Muskeltrainer zeigt das Entstehen eines solchen Tones, dass die einstellbare "Mindestgegenkraft" für das Training erreicht wurde.

Die entstehenden unterschiedlichen Töne sind für eine Auswertung bzw. Anleitung unter der Therapie bzw. des Trainings hinsichtlich ihrer Lautstärke, ihrer Dauer und in ihrem zeitlichen Verhältnis zur Ausatmung wichtig.

Insbesondere wenn der Schwingkörper aus einem Haltekörper mit einem quaderförmigen Kastenprofil ausgebildet ist, an dem in den beiden seitlichen parallel zur vertikal verlaufenden Seitenwand die Öffnungen für den Luftdurchtritt eingearbeitet sind, können die Zungen ohne Einfluss der vorherrschenden Schwerkraft alleine durch die Ein- oder Ausatmungstätigkeit des Patienten in Oszillation versetzt sein. Darüber hinaus strömt die Luft nicht senkrecht auf die Zungen, sondern streicht vielmehr zwischen den freien Enden der Zungen und der Ober- bzw. Unterseite des Haltekörpers im Bereich der Öffnungen entlang, wodurch eine Vibration an den freien Enden der Zungen entsteht, durch die die gewünschte und erforderliche Oszillation der Luftdrücke in dem Hohlkörper entsteht.

Diese Vibrationen erzeugen zudem eine Akustik, da die jeweilige Zunge als Klangkörper anzusehen ist. Alleine diese Vibration der Zunge und der damit verbundene Klang dienen dem Patienten dazu, das Ein- bzw. Ausatmungsverhalten zu erkennen. Wenn nämlich der erzeugte Klang zu tief, zu hoch oder die Lautstärke zu niedrig sein sollte, erkennt der Patient sofort, also während des Einatmungs- oder Ausatmungsvorganges, dass diese Atmungstätigkeiten den gewünschten Therapie- oder Trainingszweck nicht erfüllen und kann folglich unverzüglich seine Lungentätigkeit anpassen bzw. verändern.

Darüber hinaus kann die eingesaugte Umgebungsluft durch die in den Stenosen eingearbeiteten Durchgangsöffnungen variabel verändert werden, da die Querschnittsflächen der jeweiligen Durchgangsöffnung an der Stenose unterschiedlich groß sind und da die Stenose drehbar in dem Hohlkörper gelagert ist, sodass der Patient - auch während der Benutzung des Atemwegstherapiegerätes - durch Verdrehen der Stenose die entsprechende eingesaugte oder ausgepresste Luftmenge individuell einstellen kann. Der an den Zungen entstehende Klang aufgrund der erzeugten Vibrationen und der entsprechenden Luftflüsse erlauben dem Patienten zudem unverzüglich eine Kontrolle, inwieweit die gewählten Einstellungen den gewünschten Therapie- oder Trainingszweck erfüllen.

Unterschiedliche Stenosen stehen zur Verfügung, die schnell und unkompliziert angebracht werden können.

Da der Atemwegswiderstand definiert ist als Druckänderung dividiert durch Flussänderung, muss eine für den Patienten erkennbare Kennzeichnung der Erreichung des gewünschten Druckes bestehen, da sonst durch unterschiedliche Flüsse bei identischer Stenose unterschiedliche Drucke vorhanden sind und damit eine unterschiedliche Trainingslast entsteht.

Der Schwingungsbeginn einer Stimmzunge ist durch deren Steifigkeit und Masse bedingt. Gleiche Stimmzungen haben bei definierter Strömung den identischen Schwingungsbeginn. Die unterschiedlichen Durchmesser von Stenosen haben bei gleicher Strömung unterschiedliche Drucke zur Folge. Die Kombination einer Stimmzunge mit Nachschaltung einer Stenose definierten Durchmessers erzeugt damit bei Schwingungsbeginn der Zunge ein reproduzierbares definiertes Druckintegral. Dies kann akustisch wahrgenommen und damit aufgezeichnet werden. Gegendieses Druckintegral muss die Atemmuskulatur arbeiten. Darüber wird sie definiert und reproduzierbar trainiert.

Von der Steifigkeit und der Länge der Zunge sowie der Verbauungswinkel hängt die Kraft ab, die notwendig ist, um die Zunge zum Schwingen zu bringen und einen Ton zu erzeugen. Wenn diese Kraft bekannt ist, kann bei vorgegebener Stenose der Widerstand, der erreicht wird, exakt bestimmt werden. Da die Steifigkeit der Zunge sich nicht ändert, ist der Druck, bei dem diese zu schwingen beginnt, stets identisch. Somit lässt sich für alle Stenosen deren "kritischer Druck" und damit der überwundene Widerstand genau bestimmen.

Neben der Änderung des Einbauwinkels kann auch die Auswahl der unterschiedlichen Stenosen, zu einem unterschiedlichen Druck führen, bei dem die Zunge zu schwingen beginnt oder die Durchgangsöffnung der Stenose offen ist.

Durch die entstehenden Druckschwankungen infolge der Schwingungen der Zunge werden die Bronchien erweitert und der Bronchialschleim, da er thixotrop ist, von den Wänden abgeschert sowie verflüssigt.

Bei Verwendung einer aufschlagenden Zunge als "Druckerzeuger und -anzeiger" ohne Stenose entsteht durch die Schwingung ein Druckbild, das dem dynamischen PEP (zero-to-peak; peak-to-zero) gleichkommt, das heißt der Druck steigt von 0 auf ein Maximum, um dann sofort wieder auf 0 zu fallen. Neben der Länge der Stimmzunge ist auch deren Form für ein bestimmtes Druckbild verantwortlich. Je länger und schwerer bzw. steifer die Zunge ist, umso höher ist auch der Initialdruck. Die Zunge muss genügend Platz in der Verbauung haben, damit der Lösabstand eingehalten werden kann, um die Zunge zum Aufschwingen zu bekommen.

Je länger die Zunge ist, umso mehr Abstand benötigt diese. Für die dynamische Druckform werden daher lange und schwere Stimmzungen verarbeitet. Die entstehende Druckform wird zur Bewegung des Bronchialschleimes, zum Abscheren und Verflüssigen, benötigt.

Wird statt der aufschlagenden Zunge eine durchschlagende Zunge benutzt, die ebenfalls bei Tonerzeugung das Erreichen eines bestimmten Druckes anzeigt, entspricht das Druckschwankungsbild hier einem kombinierten PEP. Die Druckschwankung setzt sich auf einen dauerpositiven Druck auf.

Durch die Verwendung von zwei unterschiedlichen Schwingkörpern, kann dann zudem rein akustisch zwischen In- und Exspiration unterschieden werden. Diese Unterschiede können mithilfe einer App bzgl. Häufigkeit, Länge und OPIP bzw. OPEP-Anwendung (unterschiedliche Oberschwingungen) aufgenommen, ausgewertet und dokumentiert werden.

Eine zusätzliche variable Stenose kann zwischen Mundstück und der Zungen führenden Röhren geschaltet werden.

Mittels der erfindungsgemäßen Anordnung von unterschiedlich ausgestalteten Schwingkörpern in einem Hohlkörper, der beispielsweise auch mit einem Inhalationsgerät oder auch in ein Schlauchsystem eines Beatmungsgerätes eingesetzt ist, kann folglich jeder Zustand eines Patienten berücksichtigt sein. Bei künstlich beatmeten Patienten kann das erfindungsgemäß beschriebene Atemwegstherapiegerät in den Beatmungskreislauf ein- und ausgeschaltet werden. So kann der Bronchialschleim gelöst und verflüssigt werden sowie die Atemmuskulatur leicht trainiert werden.

In der Zeichnung sind acht Ausführungsvarianten eines Atemwegstherapiegeräts dargestellt, die nachfolgend näher erläutert sind. Im Einzelnen zeigt:
- Figur 1: ein erstes Ausführungsbeispiel, eines Atemwegstherapiegeräts, das aus einem Hohlkörper und einem an diesem befestigten Mundstück besteht und durch das ein Patient Atemluft aus der Umgebung einsaugt, wobei das freie Ende des Hohlkörpers mit einer Stenose, in der drei Durchgangsöffnungen mit unterschiedlichen Öffnungsquerschnitten eingearbeitet sind und verkleinert ist, wobei ein erster Schwingkörper in einem Durchgangskanal des Hohlkörpers angeordnet ist, in Seitenansicht,
- Figur 2a: eine Vergrößerung des ersten Schwingkörpers in Form einer Halteplatte, in die eine rechteckförmige Öffnung eingearbeitet ist, die von einer Zunge abgedeckt ist, im angehobenen Zustand gemäß Figur 1,
- Figur 2b: die Halteplatte gemäß Figur 2b mit abgesenkter Zunge, durch die die Austrittsöffnung verschlossen ist,
- Figur 2c: eine Verlängerung der Öffnung mit einer die Öffnung in beide Richtungen durchschlagende Zunge,
- Figur 3a: ein zweites Ausführungsbeispiel eines Atemwegstherapiegeräts, das sowohl zur Einatmung als auch zur Ausatmung verwendet werden kann, mit zwei ineinander mündenden Hohlkörpern, deren jeweilige freie Stirnseiten mit einer der Stenosen verschlossen ist und mit drei im Inneren der Hohlkörper angeordneten Schwingkörpern in Form einer Halteplatte mit Zunge bzw. eines Ventils, während des Einatmungsvorganges,
- Figur 3b: das Atemwegstherapiegerät gemäß Figur 3a, während des Ausatmungsvorgangs,
- Figur 4: ein drittes Ausführungsbeispiel eines Atemwegstherapiegeräts, in dem die Schwingkörper schräg zur jeweiligen Symmetrieachse der Hohlkörper angeordnet sind oder orthogonal zu diesen verlaufen,
- Figur 5: das Atemwegstherapiegerät gemäß Figur 1, an dessen Schwingkörper Sensoren und Mikrofone angeschlossen sind, die mit einem externen Gerät kommunizieren,
- Figur 6a: ein viertes Ausführungsbeispiel eines Atemwegstherapiegeräts, in dessen Hohlkörper eine Trennwand vorgesehen ist, durch die der Hohlkörper in zwei Durchgangskanäle unterteilt ist, durch die die Atemluft beim Einatmen strömt, in denen jeweils mindestens eine der beiden Schwingkörper eingebaut ist,
- Figur 6b: das Atemwegstherapiegerät gemäß Figur 6a, durch die die ausgeatmete Atemluft durch den zweiten Durchgangskanal in die Umgebung zurückgedrückt ist,
- Figur 7: ein fünftes Ausführungsbeispiel eines Atemwegstherapiegerätes in Weiterbildung der Ausführungsversion gemäß den Figuren 6a und 6b mit zwei in dem jeweiligen Durchgangskanal vorgesehenen Schwimmkörpern,
- Figur 8: ein sechstes Ausführungsbeispiel eines Atemwegstherapiegeräts mit zwei in den jeweiligen Durchgangskanälen des Atemwegstherapiegeräts gemäß Figur 6a angeordneten Schwimmkörpern in orthogonaler bzw. schräger Anordnung, und
- Figur 9: ein siebtes Ausführungsbeispiel eines Atemwegstherapiegerätes gemäß Figur 1, in den ein quaderförmiges Kastenprofil als Haltekörper für die Zungen in den Hohlkörper eingesetzt ist, in Draufsicht,
- Figur 10: das Atemwegstherapiegerät gemäß Figur 9 mit zwei Luftströmungen, durch die der Ein- und Ausatmungsvorgang sowie das Schwingverhalten der Zungen dargestellt ist,
- Figur 11a: eine der in den Hohlkörper des Atemwegstherapiegerätes gemäß den Figuren 1 bis 10 eingesetzten Stenosen mit unterschiedlich groß ausgestalteten Luftquerschnittsflächen als erste Alternative,
- Figur 11b: eine zweite Alternative für eine Stenose gemäß Figur 11a,
- Figuren 12a bis 12c ein: Teilquerschnitt des Haltekörpers gemäß den Figuren 9 und 10 mit unterschiedlichen Schwingverhalten der an diesem angebrachten Zungen,
- Figur 13: ein Luftströmungsdiagramm für unterschiedliche Stenosen und Zungen gemäß eines der Atemwegstherapiegeräte gemäß den Figuren 1 bis 10,
- Figur 14: eines der Atemwegstherapiegeräte gemäß den Figuren 1 bis 10 mit einem Adapter in Y-Form, durch den die offene Stirnseite des Hohlkörpers in zwei Lufteintritts-bzw. Austrittsöffnungen aufgeteilt ist, und
- Figur 15: eines der Atemwegstherapiegeräte gemäß den Figuren 1 bis 10 in einer nasalen Anwendung mit daran angeschlossener Maske.

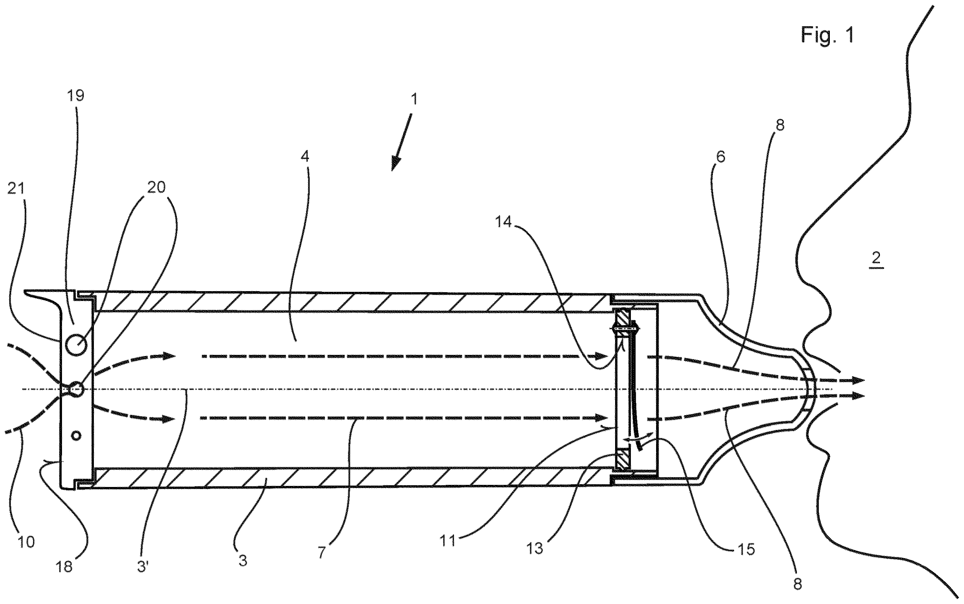

In Figur 1 ist ein Atemwegstherapiegerät 1 abgebildet, durch das ein Patient 2 seine Atemmuskulatur zur Schleimlösung in den Bronchien und/oder seine Lungenfunktion verbessern kann. Das Atemwegstherapiegerät 1 besteht aus einem Hohlkörper 3, der beispielsweise als Rohrstück ausgestaltet ist. An einem der freien Enden des Hohlkörpers 3 ist ein Mundstück 6 vorgesehen, das mit dem Patienten 2 verbunden werden kann, sodass der Patient 2 durch seine Atemmuskulatur durch das Mundstück 6 Umgebungsluft 10 einsaugen kann.

Die dem Mundstück 6 gegenüberliegende Stirnseite des Hohlkörpers 3 ist mit einer Stenose 18 verschlossen. In die Mantelfläche 19 der Stenose 18 sind drei Durchgangsöffnungen 20 mit unterschiedlich groß bemessenen Durchmessern oder Querschnittsflächen eingearbeitet. Sobald der Patient 2 durch das Mundstück 3 Umgebungsluft 10 einsaugt, strömt diese durch die Durchgangsöffnungen 20 in das Innere des Hohlkörpers 3, der folglich als Durchgangskanal 4 für die eingesaugte Umgebungsluft 10 dient. Die im Inneren des Hohlkörpers 3 entstehenden Luftströmungen sind dabei mit den Bezugsziffern 7 und 8 gekennzeichnet, wobei die Bezugsziffer 7 die einzuatmende Umgebungsluft und die Bezugsziffer 8 die ausgeatmete Luft des Patienten darstellt. Aufgrund der Anzahl und dem vorhandenen Durchmesser der Durchgangsöffnungen 20 benötigt der Patient 2 bereits eine gesteigerte Atemkraft, um die Umgebungsluft 7 überhaupt in den Durchgangskanal 4 einzusaugen.

Da die Atemluft möglichst vibrierende oder oszillierende Drücke aufbauen soll, ist unmittelbar vor dem Mundstück 6 im Durchgangskanal 4 ein erster Schwingkörper 11 angeordnet.

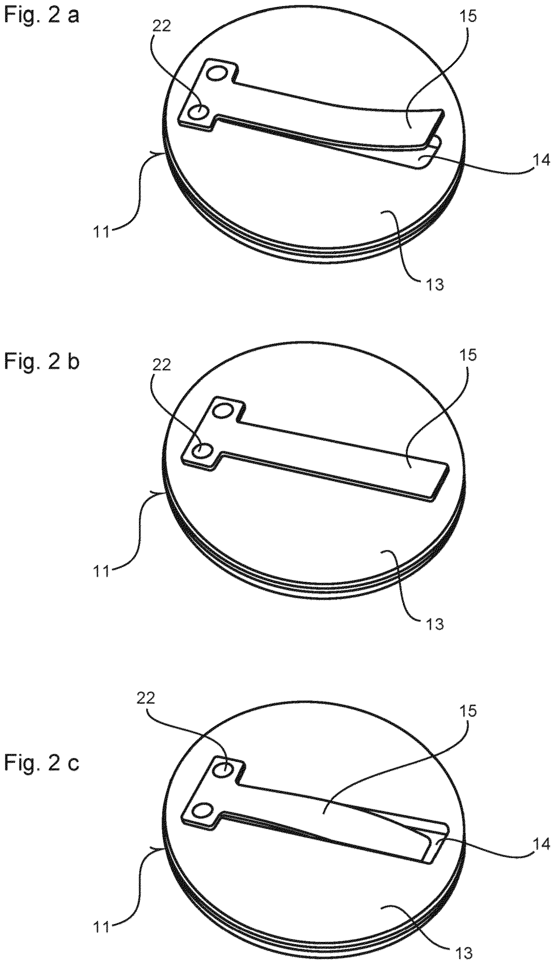

Gemäß Figur 2a umfasst der erste Schwingkörper 11 einen plattenförmigen Haltekörper 13, in den eine rechteckförmige Öffnung 14 eingearbeitet ist. Die Öffnung 14 ist von einer Zunge 15 bereichsweise im unbetätigten Zustand verschlossen. Ein freies Ende der Zunge 15 ist dabei mittels zweier Schrauben 22 an dem Haltekörper 13 befestigt. Die Außenkontur des Haltekörpers 13 ist dabei an die Innenkontur des Durchgangskanals 4 des Hohlkörpers 3 angepasst, sodass die eingesaugte Atemluft 7 ausschließlich durch die Öffnung 14 in Richtung des Mundstückes 6 strömen kann, denn die Mantelfläche des Haltekörpers 13 liegt luftdicht an der Innenwand des Durchgangskanals 4 an. Die Zunge 15 schwingt demnach in Richtung des Mundstücks 6, sobald der Patient 2 Atemluft 7 durch das Mundstück 6 saugt. Somit ist die Zunge 15 an der freien Stirnseite, die den Schrauben 22 gegenüberliegt, durch den vom Patienten 2 aufgebrachten Einatmungsdruck angehoben und die Austrittsöffnung 14 ist bereichsweise freigegeben.

Sobald der Einatmungsvorgang durch den Patienten 2 beendet ist, federt die Zunge 15 gemäß Figur 2b auf die Oberseite des Haltekörpers 13 zurück und die Öffnung 14 wird folglich vollständig verschlossen, sodass diese luftdicht abgedichtet ist. Die von der Zunge 15 erzeugten vibrierenden oder oszillierenden Luftdruckschwankungen hängen folglich von der Biegesteifigkeit der Zunge 15 und der Masse der Zunge 15 sowie den Abmessungen des Durchmessers der Stenose 18 bzw. der Öffnung 14 ab.

In Figur 2c ist gezeigt, dass die Öffnung 14 verlängert werden kann, sodass die Länge der Zunge 15 kleiner bemessen ist als die Länge der Öffnung 14. Dies bedeutet, dass zwischen dem freien Ende der Zunge 15 und der Innenseite der Öffnung 14 ein Luftspalt entsteht und die Zunge 15 in beide Richtungen durch die Öffnung 14 schwingen oder tauchen kann. Folglich schwingt die Zunge 15 durch die Öffnung 14 und somit kann das Atemwegs-therapiegerät 1 gemäß Figur 1 bei einer derartigen Ausgestaltung des Schwingkörpers 11 sowohl zum Einatmen als auch zum Ausatmen verwendet werden.

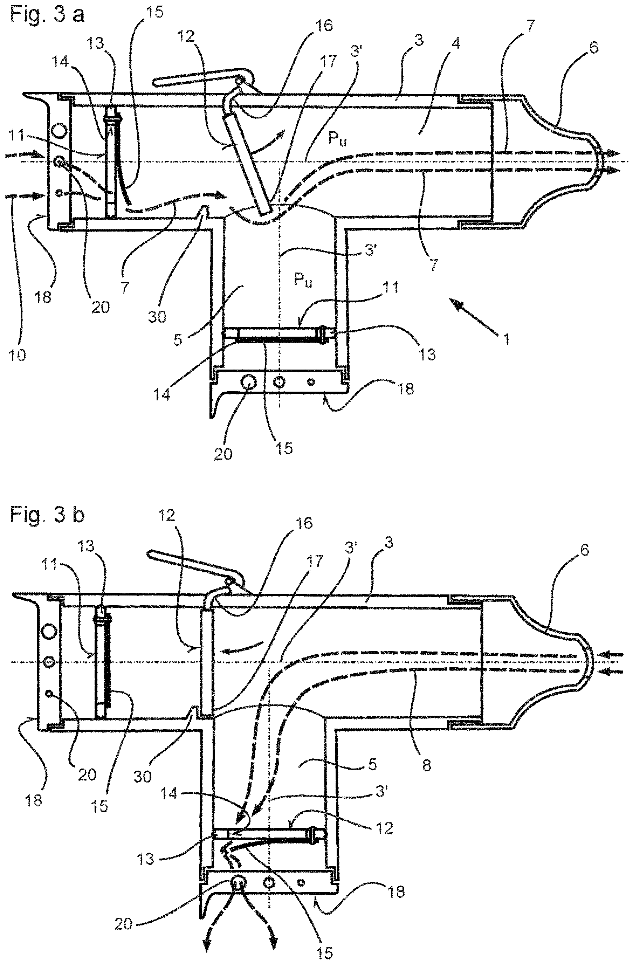

In den Figuren 3a und 3b ist eine andersartige Ausgestaltung des Atemwegstherapiegeräts 1 zu entnehmen, das nunmehr zwei miteinander verbundene Hohlkörper 3 aufweist, deren jeweilige mit der Bezugsziffer 3' gekennzeichneten Symmetrieachsen senkrecht zueinander verlaufen. Die jeweiligen freien Stirnseiten der beiden Hohlkörper 3 sind mittels der Stenose 18 verschlossen. In dem ersten Durchgangskanal 4 sind zwei Schwingkörper 11 und 12 angeordnet.

Der erste Schwingkörper 11 umfasst den Haltekörper 13 mit der Öffnung 14, die von der Zunge 15 gemäß Figur 2a und 2b verschlossen bzw. teilweise freigegeben ist. Darüber hinaus ist in dem Durchgangskanal 4 ein zweiter Schwingkörper 12 angeordnet, dessen erste Stirnseite mit dem Hohlkörper 3 verschwenkbar mittels eines Drehgelenkes 16 verbunden ist. Das gegenüberliegende freie Ende des Schwingkörpers 12 kann sich von einem an der Innenseite des Hohlkörpers 3 angeformten Anschlag 30 abheben und ist mit der Bezugsziffer 17 gekennzeichnet, wenn der Patient 2 Umgebungsluft 7 durch die Stenose 18 in den Durchgangskanal 4 einsaugt. Der zweite Schwingkörper 11 wirkt als eine Art Ventil.

Die Zunge 15 des ersten Schwingkörpers 11 hebt sich dabei von der Öffnung 14 ab. In dem Durchlasskanal 4 entsteht ein Unterdruck, der mit Pu gekennzeichnet ist. Da die beiden Durchgangskanäle 4 und 5 miteinander verbunden sind, entsteht im zweiten Durchgangskanal 5 der Unterdruck Pu, wodurch die Zunge 15 des ersten Schwingkörpers 11 auf den Haltekörper 13 gedrückt ist, um die Öffnung 14 zu verschließen. Die Zunge 15 ist nämlich auf der dem ersten Durchgangskanal 4 abgewandten Seite des Haltekörpers 13 angeordnet, sodass der Unterdruck Pu die Zunge 15 auf den Haltekörper 13 zieht.

Beim Ausatmen gemäß Figur 3b werden die beiden Schwingkörper 11 und 12 im ersten Durchgangskanal 4 verschlossen, denn das Ventil 16 wird gegen den Anschlag 30 beim Ausatmen durch die Atemluft 8 gedrückt; ebenso verschließt sich die Öffnung 14, da die Zunge 15 auf den Haltekörper 13 aufgrund der vorherrschenden Vorspannkraft der Zunge 15 gedrückt ist und somit auf diese aufliegt. Die ausgeatmete Atemluft 8 strömt demnach durch das Mundstück 6 in den ersten Durchgangskanal 4 und anschließend in den zweiten Durchgangskanal 5, sodass die Atemluft 8 auf die Zunge 15 des im Durchgangskanal 5 angeordneten Schwingkörper 11 auftrifft und dabei die Zunge 15 bereichsweise von der Öffnung 14 abhebt bzw. aufdrückt. Die Atemluft 8 strömt anschließend durch die in der freien Stirnseite des zweiten Hohlkörpers 3 angeordneten Durchgangsöffnung 20 der Stenose 18 in die Umgebung.

Folglich kann das Atemwegstherapiegerät 1 gemäß den Figuren 3a und 3b sowohl zum Einatmen als auch zum Ausatmen verwendet werden. Durch die zwei unterschiedlich wählbaren Stenosen 18 für Ein- und Ausatmung lässt sich die Trainingslast für diese beiden Atemmanöver individuell und getrennt einstellen.

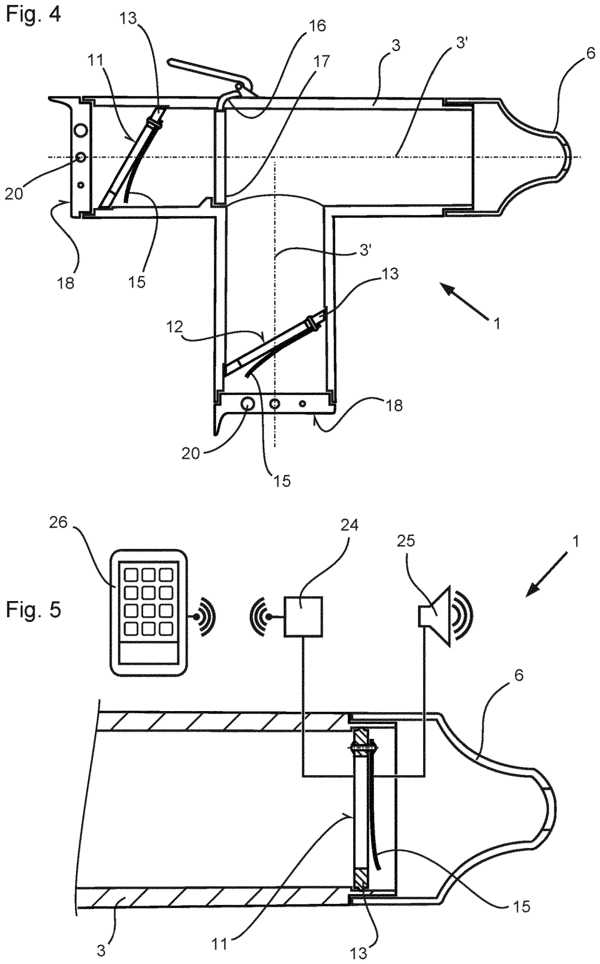

In Figur 4 ist eine andersartige Anordnung der Schwingkörper 11 in den beiden Hohlkörpern 3 dargestellt. Gemäß den Figuren 3a und 3b verlaufen die Schwimmkörper 11 senkrecht zu der Symmetrieachse 3' des jeweiligen Hohlkörpers 3 und in Figur 4 ist zu entnehmen, dass die Schwingkörper 11 in einem vorgegebenen Winkel, also geneigt zu der jeweiligen Symmetrieachse 3`, angeordnet sind.

Gemäß Figur 5 soll der Schwingkörper 11 sowohl als akustische als auch als elektronische Übertragungseinrichtung genutzt werden. Daher sind beispielsweise an der Zunge 15 zugeordnete Lautsprecher 25 oder elektrische Sensoren 24 vorgesehen. Die Lautsprecher 25 übertragen dabei einen Ton, sodass der Patient 2 während der Benutzung des Atemtrainers (Inspiration) bzw. Atemwegstherapiegeräts (Expiration) 1 akustisch wahrnehmen kann, ob seine Atemmuskulatur die Zunge 15 ausreichend in Schwingung versetzt und wie lange dieser den oszillierenden Widerstand, also die vorgegebene Trainingslast halten kann.

Die Sensoren 24 kommunizieren beispielsweise über eine WLAN-Verbindung mit einem externen Gerät 26. Dabei kann das Gerät 26 die Benutzungsdauer, die verwendeten Frequenzen der Zunge 15 und die Anzahl der Atemübungen des Patienten 2 aufzeichnen, sodass diese beispielsweise von einem Arzt nach einer bestimmten Zeitspanne ausgelesen und ausgewertet werden können.

Werden dann noch zwei verschiedene Zungen 15 eingebaut, kann direkt zwischen dem Training der Einatemmuskulatur inklusive der entsprechenden Trainingslast bzw. bei der Ausatmung die Dauer inklusive der resistiven und thresholdgetriebenen Therapiehöhe unterschieden und aufgezeichnet werden. Für diese Aufzeichnungen kann auch über eine APP ein herkömmliches Handy genutzt werden, so dass Lautsprecher und Mikrofon überflüssig werden könnten.

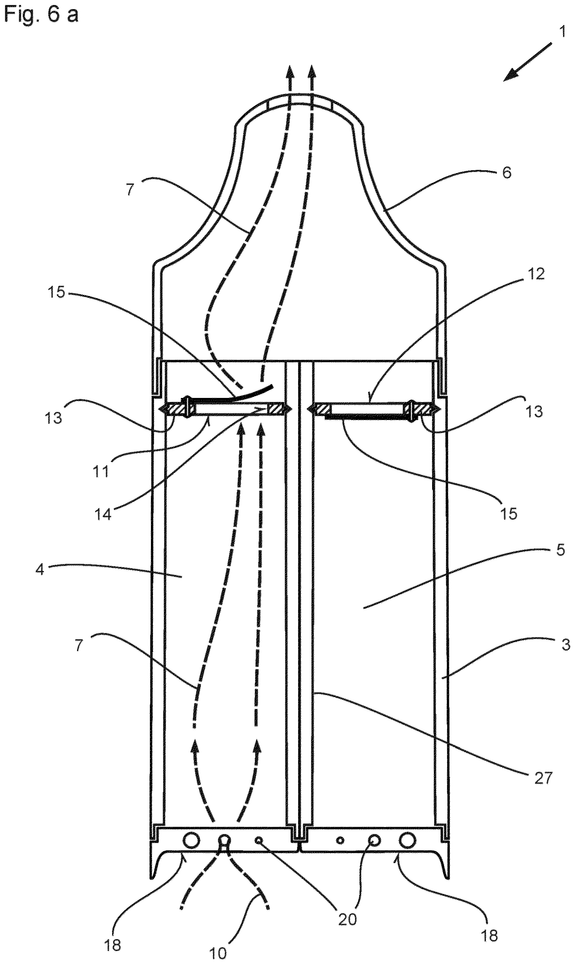

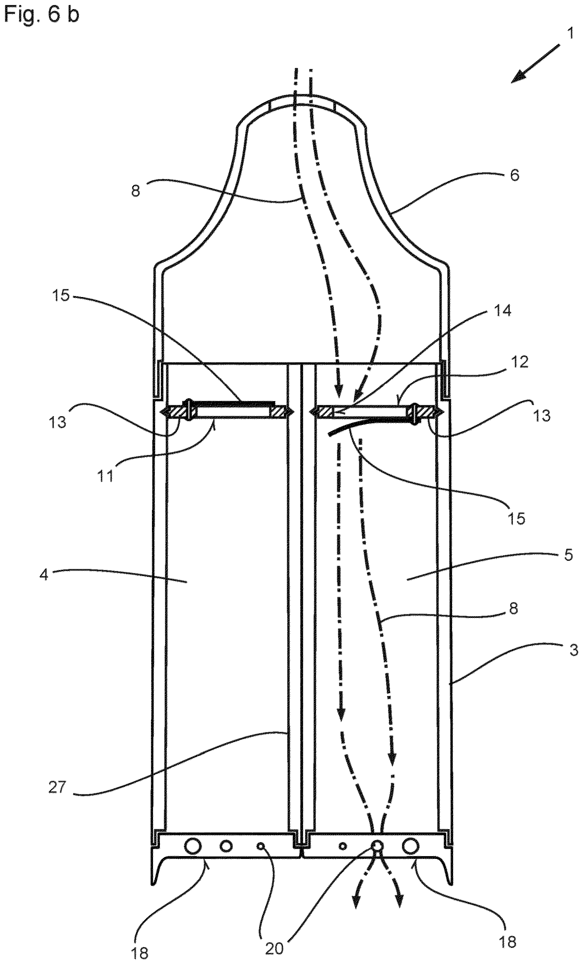

In den Figuren 6a und 6b umfasst der Atemtrainer bzw. das Atemwegstherapiegerät 1 einen Hohlkörper 3, in dem eine Trennwand 27 zur Bildung von zwei luftdicht voneinander getrennten Durchgangskanälen 4 und 5 gebildet ist. Die jeweiligen freien Stirnseiten des Hohlkörpers 3 sind mittels der Stenose 18 teilweise verschlossen. In jedem der Durchgangskanäle 4 und 5 ist einer der Schwingkörper 11 angeordnet. Gemäß Figur 6a ist der im Durchgangskanal 4 vorgesehene Schwimmkörper 11 geöffnet, sobald der Patient Atemluft 7 durch den Durchgangskanal 4 saugt, da die Zunge 15 in Richtung des Mundstückes 6 durch die eingesaugte Atemluft 7 aufgedrückt ist. Die Zunge 15 ist auf der dem Mundstück 6 zugewandten Oberseite der Halteplatte 13 angeordnet.

Gemäß Figur 6b öffnet sich die Zunge 15 im Durchgangskanal 5, sobald der Patient 2 ausatmet, da die Atemluft 8 zunächst die Zunge 15 des Schwingkörpers 11 im Durchgangskanal 4 auf den Haltekörper 13 drückt und folglich der Durchgangskanal 4 verschlossen ist und die Atemluft 8 die Zunge 15 des im Durchgangskanal 5 angeordneten Schwingkörper 11 aufdrückt. Die Zunge 15 ist auf der dem Mundstück 6 abgewandten Oberseite des Haltekörpers 13 angeordnet.

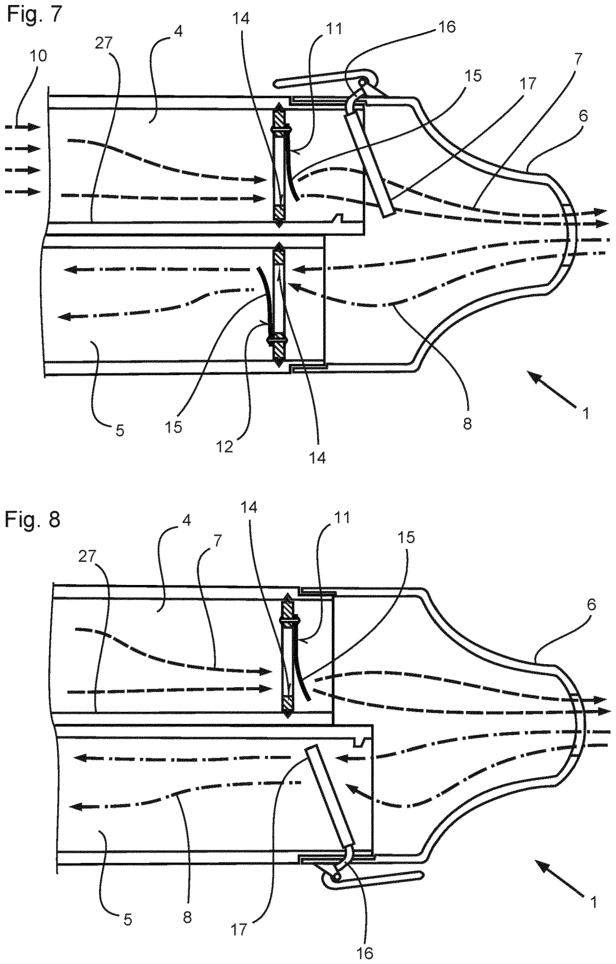

In Figur 7 ist eine Kombination aus erstem Schwingkörper 11 mit durchschlagender Zunge 15 und zweitem Schwingkörper 12 abgebildet. Der Durchgangskanal 4 ist dabei sowohl mittels des Schwingkörpers 11 und des Schwingkörpers 12 beim Einatmen geöffnet und beim Ausatmen sind diese durch die Atemluft 8 verschlossen, sodass die Atem luft 8 den Schwingkörper 11 im Durchgangskanal 5 aufdrückt.

Es ist möglich, eine Kombination aus Inhalationsgerät / Vernebler 36 mit zweitem Schwingkörper 12 zu benutzen. Der Durchgangskanal 4 ist dabei mittels des Schwingkörpers 12 beim Einatmen geöffnet und beim Ausatmen ist dieser durch die Atemluft 8 verschlossen, so dass die Atemluft 8 den Schwingkörper 11 im Durchgangskanal 5 aufdrückt.

In Figur 8 ist gezeigt, dass der Durchgangskanal 4 mittels eines Schwingkörpers 11 und der Durchgangskanal 5 mittels eines Schwingkörpers 12 verschlossen ist. Wahlweise können diese Kombinationen entsprechend ausgetauscht sein und die Ausrichtung der Schwingkörpers 11 und 12 bezogen auf die Symmetrieachse 3' des Hohlkörpers 3 können senkrecht oder geneigt zu dieser verlaufen.

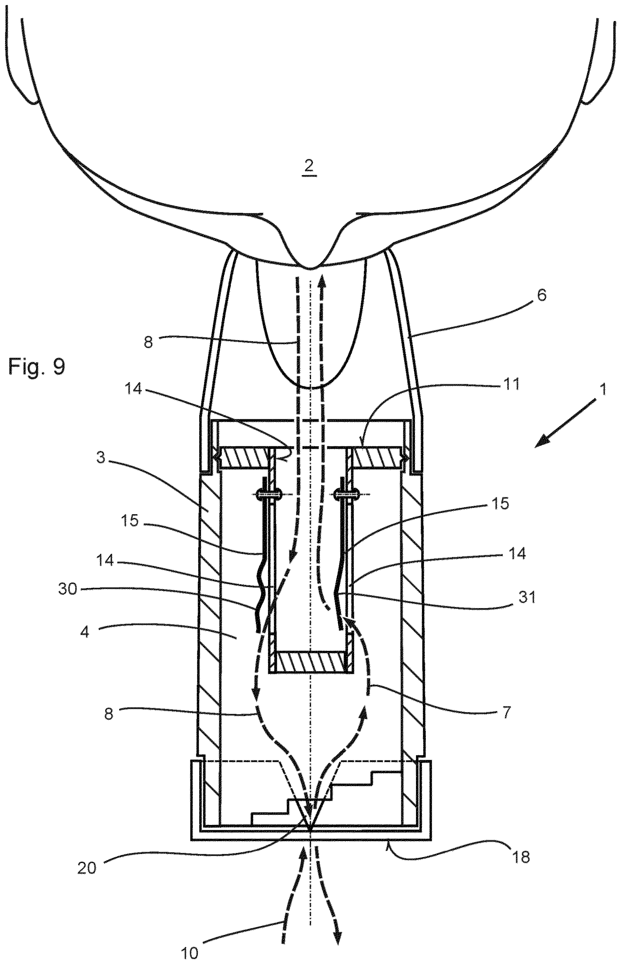

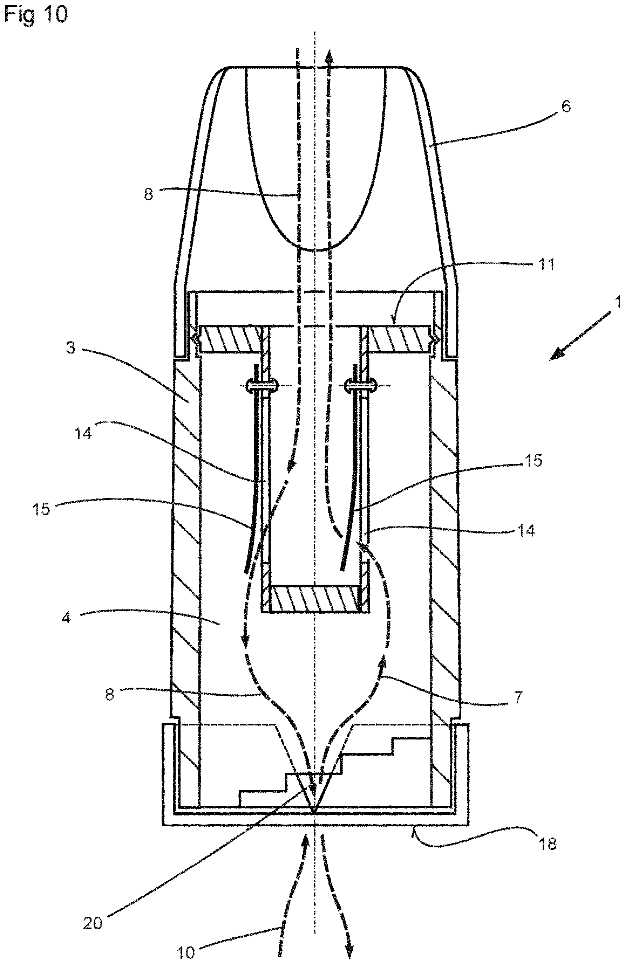

In den Figuren 9 und 10 ist eine weitere alternative Ausgestaltung des Atemwegstherapiegerätes 1 zu entnehmen. Dabei besteht der Schwingkörper 11 zunächst aus dem Haltekörper 13, der im Querschnitt T-förmig ausgestaltet ist. Dabei sind die freien Enden des Haltekörpers 13 an der Innenseite des Hohlkörpers 3 befestigt, durch die eine der Öffnungen 14 eingeschlossen ist. Diese Öffnung 14 verläuft demnach gegenüberliegend zu dem Mund-Nasenstück 6 bzw. mündet in dieses, sodass die Luftströmungen 7, 8 im Wesentlichen senkrecht durch diese Öffnungen streichen.

Darüber hinaus ist das Atemwegstherapiegerät 1 mit einem quaderförmigen Kastenprofil als Bestandteil des Haltekörpers 13 ausgerüstet. Das quaderförmige Kastenprofil schließt demnach einen Hohlraum ein, der mit der Öffnung 14 kommuniziert bzw. in diese mündet. Zwei gegenüberliegende Seitenflächen des Kastenprofils 13 weisen zwei Öffnungen 14 auf, an denen außenseitig bzw. innenseitig die Zungen 15 angebracht sind. Wenn das Atemwegstherapiegerät 1 benutzt ist, verlaufen die beiden Seitenflächen des Haltekörpers 13 parallel zu der Vertikalen, sodass Eigengewichtskräfte der Zungen 15 deren Schwingverhalten nicht beeinflussen; vielmehr ist das Schwingverhalten der Zungen 15 ausschließlich durch die erzeugte Luftbrücke, Luftschwankungen aufgrund der an der Stenose 18 vorgesehenen Durchgangsöffnungen 20 und aufgrund der Materialbeschaffenheit der Zungen 15 bzw. der Geometrie in der Öffnung 14 beeinflusst. Die Zungen 15 bewegen sich somit in einer vertikalen Ebene und können sowohl beim Einatmen als auch beim Ausatmen des Patienten 2 in entsprechende Schwingungen oder Oszillationen versetzt sein. Das Strömungsverhalten im Inneren des Hohlkörpers 3 und im Bereich der Öffnungen 14 und der Zungen 15 ist schematisch mit den Bezugsziffern 10, 7 sowie 8 dargestellt. Wenn daher der Patient 2 einatmet, wird die Umgebungsluft 10 durch eine der Durchgangsöffnungen 20 der Stenose 18 in den Durchgangskanal 4 des Hohlkörpers 3 eingesaugt und die innenseitig angebrachte Zunge 15 schwingt auf, sodass die eingeatmete Umgebungsluft 7 durch die Öffnung 14 und den Luftspalt zwischen der Zunge 15 und dem Haltekörper 13 durch die dem Patienten 2 zugewandte Öffnung 14 strömt und von diesem eingeatmet werden kann. Beim Ausatmen strömt die Atem luft 8 in das Innere des Kastenprofils des Haltekörpers 13 und drückt die innenseitig angebrachte Stimmzunge 15 zu, sodass deren Öffnung 14 verschlossen ist; wohingegen die außenseitig angelenkte Zunge 15 nach außen aufschwingt und die Atemluft 8 durch den von dieser freigegebenen Luftspalt in den Durchgangskanal 4 des Hohlkörpers 3 und aus diesem durch die Durchgangsöffnung 20 der Stenose 18 nach außen gelangt.

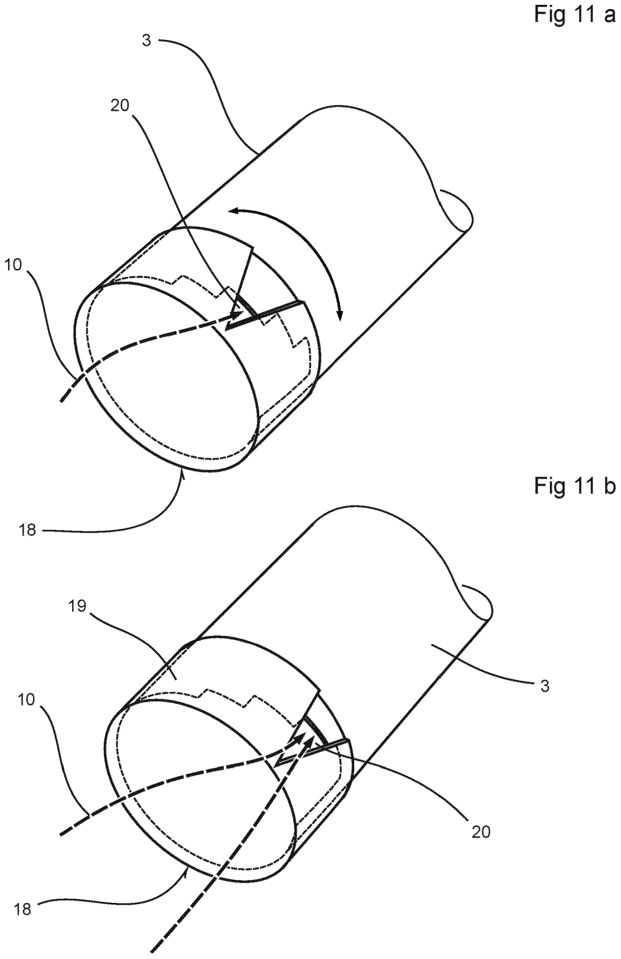

In den Figuren 11a und 11b sind zwei unterschiedliche Ausführungsbeispiele einer der üblichen Stenosen 18 mit unterschiedlich groß ausgestalteten Durchgangsöffnungen 20 dargestellt. Die jeweilige Stenose 18 ist dabei in dem Hohlkörper 3 drehbar gelagert und deren Mantelfläche 19 weist vier unterschiedlich ausgestaltete Durchgangsöffnungen 20 auf, die durch Verdrehen der Stenose 18 relativ zu dem Hohlkörper 3 derart positioniert werden können, dass die entsprechende Einlassöffnung verkleinert oder vergrößert ist. Die entsprechende Auswahl der Querschnittsfläche der jeweiligen Durchgangsöffnung 20 führt demnach dazu, dass der Patient 20 mit mehr oder weniger Ansaugdruck die Umgebungsluft 10 in den Durchgangskanal 4 des Hohlkörpers 3 einsaugen bzw. die Atemluft 8 aus dem Durchgangskanal 4 in das Freie auspressen muss. Die entsprechend eingestellte Querschnittsfläche der Durchgangsöffnungen 20 üben darüber hinaus einen erheblichen Einfluss auf das Schwingverhalten der jeweiligen Zunge 15 aus, denn bei höherem Innendruck im Durchgangskanal 4 verändert sich das Schwingverhalten der jeweiligen Zunge 15.

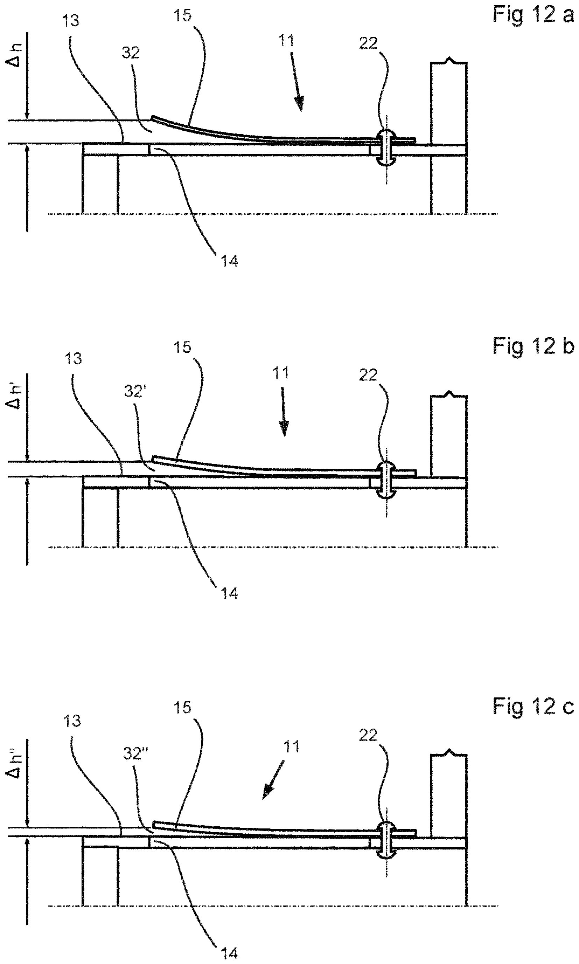

Dieses Schwingverhalten der Zungen 15 ist in den Figuren 12a, 12b sowie 12c gezeigt und verdeutlicht. Zunächst unterscheiden sich die Auslenkungen der Zungen 15 dadurch, dass der Abstand Δ h zwischen der Unterseite der Zunge 15 und der Oberseite des Haltekörpers 13 unterschiedlich groß ausgebildet sind. Diese Abstandsveränderung ist mit den Bezugsziffern 32, 32'sowie 32"wiedergegeben. Diese Abstandsveränderungen 32, 32' oder 32" hängen zunächst von dem verwendeten Werkstoff der Zunge 15 ab, die aus einem biegeelastischen Metall hergestellt sind. Mit unterschiedlichen Werkstoffeigenschaften verändert sich das Schwingverhalten der Zunge 15. Ferner kann das Schwingverhalten der Zungen 15 auch durch die Geometrie in der Öffnungen 14 und der Dicke der Zungen 15 beeinflusst sein.

Es hat sich zudem gezeigt, dass bei bestimmten Paarungen von Werkstoffen, Geometrien und Druckverhältnissen im Durchgangskanal 4 bzw. in dem Hohlkörper 13 eine akustisch wahrnehmbare Tonfolge durch das Schwingverhalten der Zungen 15 entsteht. Diese Akustik dient dazu, dass der Patient 2 exakt feststellen kann, ob sein Atemverhalten den gewünschten Trainings- oder Therapiezweck erfüllt. Wenn nämlich die sich einstellende Akustik schwach ist oder Misstöne erzeugt sind, dann erkennt der jeweilige Patient 2 an der vorherrschenden Akustik, dass das Atemverhalten nicht korrekt ist und somit Veränderungen an der Stenose 18 oder dem Haltekörper 13 bzw. den eingesetzten Zungen 15 vorzunehmen sind.

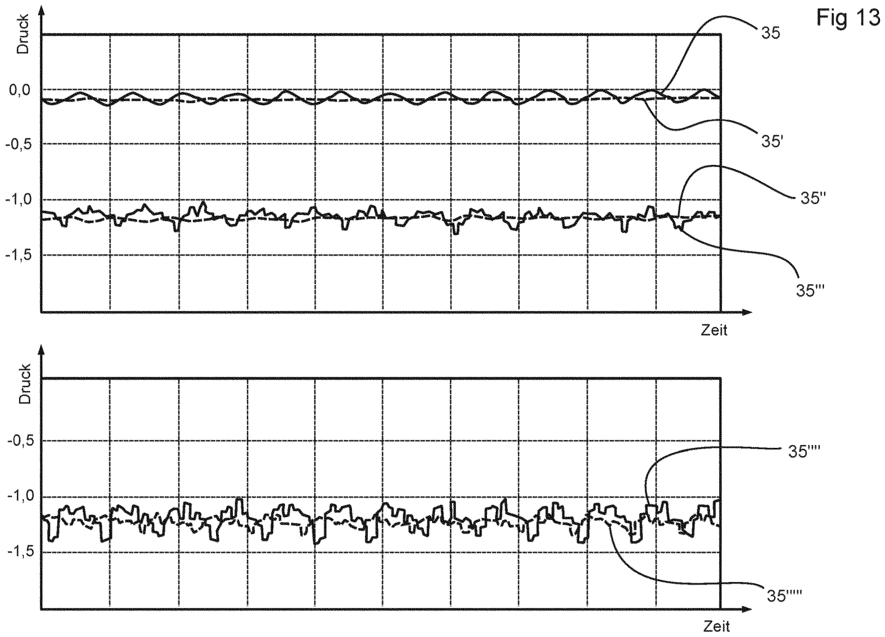

In Figur 13 sind drei unterschiedliche Schwingverhalten schematisch wiedergegeben. Das akustische Verhalten des Atemwegstherapiegerätes 1 ist somit durch den vordefinierten Innendruck im Durchgangskanal 4 und den Fluss durch die Öffnungen 14, durch die das Schwingverhalten der Zungen 15 beeinflusst und vorgegeben ist, abgebildet. Gemäß den Figuren 12a, 12b und 12c verändert sich der Abstand 32, 32' bzw. 32", wodurch die Durchflussmenge der eingeatmeten Umgebungsluft 7 bzw. der Atem luft 8 entsteht. Je größer demnach dieser Abstand 32, 32` oder 32"ist, desto mehr Luftvolumen fließt durch die Öffnungen 14 bzw. ein Über- oder Unterdruck in dem Durchgangskanal 4 wird erzeugt bzw. ist notwendig, um die Zunge 15 zum Schwingen zu bringen. Hierbei lässt sich in Kombination mit der jeweiligen vorgeschalteten Stenose 18 der minimal für eine Tonbildung erforderliche Fluss bzw. Unter- oder Überdruck exakt einstellen.

Die in den Diagrammen der Figur 13 abgebildeten Schaubilder zeigen die Beziehungen zwischen diesen Einstellmöglichkeiten. Dabei ist auf der Abszisse die Zeit und auf der Koordinate der jeweilige Druck eingetragen, wobei 1 V einem Druck von 20 cm H₂O entspricht. Die hier exemplarisch aufgezeigten Messungen sind mit einem Unterdruck verwirklicht worden. Dies gilt jedoch in identischer Weise für den bei der Ausatmung entstehenden Überdruck. Bei diesen Messungen mit einem geringen Löseabstand 32 und der größten bzw. weitesten Querschnittsfläche der Durchgangsöffnung 20 der Stenose 18 zeigt sich eine ausgeprägte Oszillation des Schwingverhaltens der Zungen 15, die selbst bei niedrigen Drücken noch deutliche Druckschwankungen aufweist. Bei hohen Drücken sind diese Druckschwankungen erheblich ausgeprägter und bewegen sich zudem auf einem hohen Druckniveau von - 1 V. Wird jedoch die kleinste Querschnittsfläche der Durchgangsöffnung 20 an der Stenose 18 ausgewählt bzw. eingestellt, dann ändert sich das Schwingverhalten nicht und ist durch die Abmessungen sowie die Steifigkeit des verwendeten Werkstoffes der Zungen 15 vorgegeben.

Die Kombination aus verschiedenen Löseabständen 32, 32' oder 32" für die Bildung der Unter- und/oder Überdrücke beim Ein- bzw. Ausatmen, die Steifigkeit und Länge der Zungen 15 mit der eingestellten Querschnittsfläche der Durchgangsöffnung 20 der jeweiligen Stenose 18 definieren somit exakt den erforderlichen Druck, der zum Anfang einer Schwingung erforderlich ist und demnach dem Training der Atemmuskulatur sowie der Lösung der bronchialen Sekrete dient. Dieses Schwingverhalten ist zudem akustisch wahrnehmbar, und zwar sowohl von dem Patienten 2 als auch von den an den Zungen 15 angebrachten Sensoren 23 oder 24. Diese Auswahl der Einstellmöglichkeiten kann somit auf jeden Patienten 2 individuell festgelegt sein, um auf die therapieerforderlichen Parameter optimal Einfluss zu nehmen.

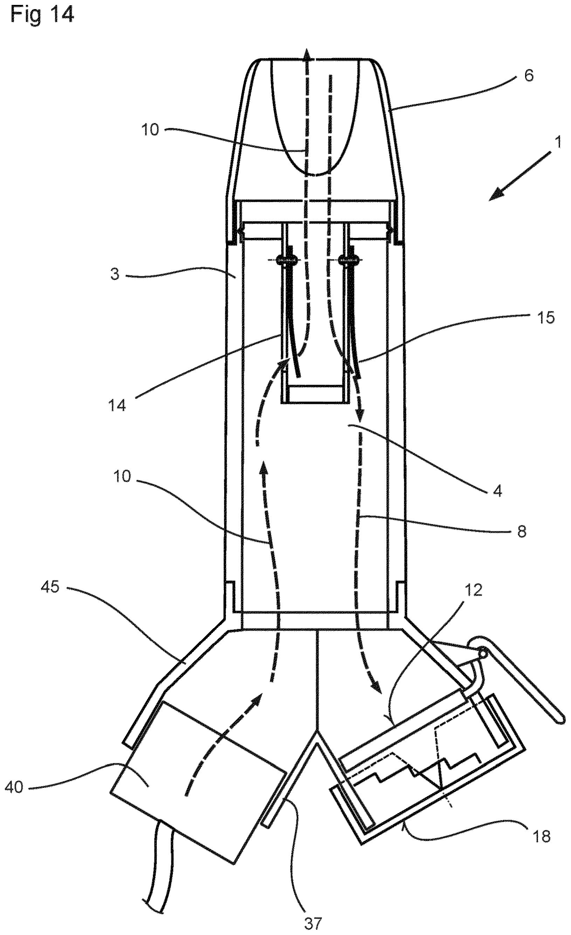

In Figur 14 ist eine konstruktive Weiterbildung des Atemwegstherapiegeräts gemäß den Figuren 9 und 10 abgebildet. Dabei ist in das freie Ende des Hohlkörpers 13 ein Adapter 37 als Weiche für die eingeatmete Umgebungsluft 10 und die Atemluft 8 zu entnehmen. Der Adapter 37 weist eine Y-Form auf, sodass dieser zwei der Umgebung zugewandte Öffnungen aufweist, in die jeweils eine der Stenosen 18 eingesetzt werden kann. Folglich kann auch der Druckzustand im Durchlasskanal 4 für den Einatmungs- bzw. Ausatmungsvorgang unterschiedlich eingestellt werden, indem entsprechend kleinere oder größere Querschnittsflächen von Durchgangsöffnungen 20 an der jeweiligen Stenose 18 ausgewählt bzw. eingestellt sind.

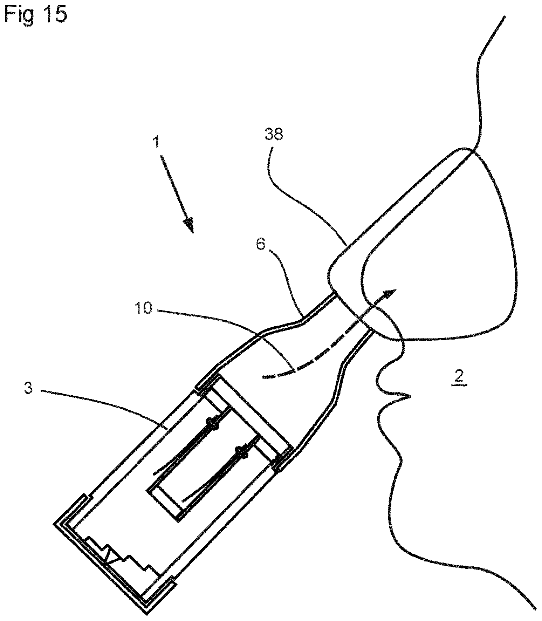

In Figur 15 ist der Anwendungsfall des Atemwegstherapiegerätes 1 für den nasalen Gebrauch gezeigt. Dabei mündet das Mundstück 6 des Atemwegstherapiegerätes in eine Maske 38, die in üblicher und bekannter Weise auf die Außenseite einer Nase des Patienten 20 aufgesetzt ist. Der Patient 20 kann demnach das erfindungsgemäße Atemwegstherapiegerät 1 auch für die Schleimlösung innerhalb des Nasenraums einsetzen.

Die Zungen 15 können aus einem biegeelastischen Werkstoff hergestellt sein. Dabei ist entscheidend, dass die Zungen 15 vibrieren und somit das akustisch wahrnehmbare Vibrieren deren Oszillation erzeugen. Folglich kann der verwendete Werkstoff aus Metall, aus einem Hartkunststoff oder aus einem Gewebe aus diesen Materialien bestehen.

## Patentansprüche

1. Atemwegstherapiegerät (1) zur Behandlung der Muskulatur von Atemwegen, Lungenfunktionen und/oder Schleimablagerungen im Lungen-, Nasen- und/oder Rachenraum eines Patienten (2), bestehend aus:
- einem Hohlkörper (3), in dem mindestens ein Durchgangskanal (4, 5) vorgesehen ist,
- einem in den Hohlkörper (3) eingesetztes Mund- oder Nasenstück (6), das mit dem Patienten (2) während der Behandlungsdauer des Atemwegstherapiegerätes (1) verbunden ist,
- und aus mindestens einem im Inneren des Hohlkörpers (3) angeordneter Schwingkörper (11, 12), der durch die Ein- und /oder Ausatmung des Patienten (2) in eine oszillierende Vibration versetzt ist,
wobei in den Hohlkörper (3) ein Haltekörper (13) eingesetzt ist, in den mindestens eine Öffnung (14) eingearbeitet ist wobei an dem Haltekörper (13) im Bereich der Öffnung (14) eine frei schwingende Zunge (15) zur Erzeugung für das menschliche Gehör akustisch wahrnehmbarer Schwingungen, die durch ihre unterschiedliche Frequenz die Inspiration von der Exspiration unterscheiden lassen, befestigt ist, durch die die Öffnung (14) des Haltekörpers (13) in Abhängigkeit von den im Inneren des Hohlkörpers (3) vorherrschenden Luftströmungen (7, 8) verschlossen oder bereichsweise freigegeben ist,
wobei die dem Mundstück (6) gegenüberliegende Stirnseite (9) des Hohlkörpers (3) offen ist, wobei in diese Stirnseite (9) eine Stenose (18) eingesetzt ist, in deren Mantelfläche (19) mindestens zwei Durchgangsöffnungen (20) vorgesehen sind, durch die Luftströmungen (7, 8) in das Innere des Hohlkörpers (3) strömen oder vice versa austreten,
wobei die Durchgangsöffnungen (20) der Stenose (18) unterschiedliche Querschnittsflächen als Luftdurchlässe für die Luftströmungen (7, 8) aufweisen und wobei die Stenose (18) in dem Hohlkörper (3) drehbar gelagert ist, derart dass durch Verdrehen der Stenose (18) eine der Durchgangsöffnungen (20) als Luftdurchlass auswählbar bzw. einstellbar ist.

2. Atemwegstherapiegerät nach Anspruch 1, wobei die Stenose (18) eine Wand (21) aufweist, durch die der Zugang in den Hohlkörper (3) in Richtung dessen Symmetrieachse (3') verschlossen ist, und wobei die Außenkontur der Stenose (18) an der Innenkontur des Hohlkörpers (3) angepasst ist.

3. Atemwegstherapiegerät nach einem der Ansprüche 1 oder 2, wobei die Durchgangsöffnungen (20) mittels eines Stopfens (23) verschließbar sind.

4. Atemwegstherapiegerät nach einem der vorgenannten Ansprüche, wobei die Zunge (15) einseitig auf der Oberfläche des Hohlköpers (13) aufliegt bzw. abhebt oder wobei die Zunge (15) durch die Öffnung (14) oszillierend Luft einströmen und/oder ausströmen lässt.

5. Atemwegstherapiegerät nach einem der vorgenannten Ansprüche, wobei ein erster und zweiter Schwingkörper (11, 12) in einer Atemrichtung und/oder in entgegengesetzter Atemrichtung angeordnet sind.

6. Atemwegstherapiegerät nach einem der vorgenannten Ansprüche,
wobei der Hohlkörper (13) als Kastenprofil ausgestaltet ist, durch den ein Hohlraum quaderförmig eingeschlossen ist, wobei an mindestens einem der Schwingkörper (11, 12) ein Sensor (24) und/oder ein Mikrofon (25) angeschlossen ist, wodurch akustische oder elektrische Signale an ein externes Gerät (26) übertragen sind,
wobei die Schwingkörper (11, 12) als akustisch wahrnehmbare Klangkörper zur Überprüfung der korrekten bzw. gewünschten Anwendung des Atemwegstherapiegerätes (1) dienen, und wobei die Öffnungen in
einer parallel in den Ventilen verlaufenden Stirnseite des Gehäuses vorgesehen sind.

7. Atemwegstherapiegerät nach einem der vorgenannten Ansprüche, wobei die Zunge (15) aus einem biegeelastischen Werkstoff, vorzugsweise aus Metall, Hartkunststoff oder einem Gewebe aus diesen Materialien, hergestellt ist.

## Claims

1. A respiratory therapy device (1) for treating the muscles of respiratory tracts, lung functions and/or mucous deposits in the lungs, nose and/or throat of a patient (2), consisting of:
- a hollow body (3) in which at least one through-channel (4, 5) is provided,
- a mouthpiece or nosepiece (6) inserted into the hollow body (3) and connected to the patient (2) over the duration of the treatment by the respiratory therapy device (1),
- and at least one oscillating body (11, 12) that is situated inside the hollow body (3) and that is set into an oscillating vibration by the inhalation and/or exhalation of the patient (2),
wherein a retaining body (13) into which at least one opening (14) is incorporated is inserted into the hollow body (3), wherein a freely oscillating tongue (15) for generating oscillations, which are acoustically perceptible to the human ear and which, due to their different frequency, enable distinguishing inspiration from expiration, is attached to the retaining body (13) in the region of the opening (14), by means of which the opening (14) of the retaining body (13) is closed or partially opened depending on the air flows (7, 8) prevailing in the interior of the hollow body (3), wherein the end face (9) of the hollow body (3) opposite the mouthpiece (6) is open, wherein a stenosis (18) is inserted into this end face (9), at least two through-openings (20) being provided in the lateral surface (19) of said stenosis, through which air flows (7, 8) flow into, or conversely, flow out of, the interior of the hollow body (3), wherein the through-openings (20) of the stenosis (18) have different cross-sectional areas as air passages for the air flows (7, 8) and wherein the stenosis (18) is rotatably mounted in the hollow body (3) in such a way that one of the through-openings (20) can be selected or adjusted as an air passage by rotating the stenosis (18).

2. The respiratory therapy device according to claim 1, wherein the stenosis (18) has a wall (21) via which the access into the hollow body (3) in the direction of its axis of symmetry (3') is closed, and wherein the outer contour of the stenosis (18) is adapted to the inner contour of the hollow body (3).

3. The respiratory therapy device according to one of claims 1 or 2, wherein the through-openings (20) are closable by means of a plug (23).

4. The respiratory therapy device according to one of the preceding claims,
wherein the tongue (15) rests on or lifts up from one side on the surface of the hollow body (13) or wherein the tongue (15) allows air to flow in and/or out through the opening (14) in an oscillating manner.

5. The respiratory therapy device according to one of the preceding claims, wherein a first and second oscillating body (11, 12) are arranged in one breathing direction and/or in the opposite breathing direction.

6. The respiratory therapy device according to one of the aforementioned claims, wherein the hollow body (13) is designed as a box profile that cuboidally encloses a cavity, wherein a sensor (24) and/or a microphone (25) is connected to at least one of the oscillating bodies (11, 12), whereby acoustic or electrical signals are transmitted to an external device (26), wherein the oscillating bodies (11, 12) serve as acoustically perceptible sound bodies for checking the correct or desired use of the respiratory therapy device (1), and wherein the openings are provided in an end face of the housing running parallel in the valves.

7. The respiratory therapy device according to one of the preceding claims,
wherein the tongue (15) is made of a flexible material, preferably of metal, hard plastic or a woven fabric made of these materials.

## Revendications

1. Un appareil de thérapie respiratoire (1) pour traiter les muscles des voies respiratoires, les fonctions pulmonaires et/ou les dépôts de mucus dans les poumons, le nez et/ou la gorge d'un patient (2), composé de :
- un corps creux (3) dans lequel au moins un canal de passage (4, 5) est prévu,
- un embout buccal ou nasal (6) inséré dans le corps creux (3) et relié au patient (2) pendant la durée du traitement par l'appareil de thérapie respiratoire (1),
- et au moins un corps oscillant (11, 12) situé à l'intérieur du corps creux (3) et soumis à une vibration oscillante par l'inspiration et/ou l'expiration du patient (2),
dans lequel un corps de retenue (13) dans lequel au moins une ouverture (14) est incorporée est inséré dans le corps creux (3), dans lequel une languette librement oscillante (15) pour générer des oscillations, qui sont acoustiquement perceptibles par l'oreille humaine et qui, en raison de leur fréquence différente, permettent de distinguer l'inspiration de l'expiration, est fixée au corps de retenue (13) dans la zone de l'ouverture (14), au moyen de laquelle l'ouverture (14) du corps de retenue (13) est fermée ou partiellement ouverte en fonction des flux d'air (7, 8) régnant à l'intérieur du corps creux (3), la face d'extrémité (9) du corps creux (3) opposée à l'embout buccal (6) étant ouverte, dans laquelle une sténose (18) est insérée dans cette face d'extrémité (9), au moins deux ouvertures de passage (20) étant prévues dans la surface latérale (19) de ladite sténose, à travers lesquelles les flux d'air (7, 8) s'écoulent vers l'intérieur du corps creux (3) ou, à l'inverse, s'en échappent, dans laquelle les ouvertures (20) de la sténose (18) ont des sections transversales différentes en tant que passages d'air pour les flux d'air (7, 8) et dans laquelle la sténose (18) est montée rotative dans le corps creux (3) de manière à ce que l'une des ouvertures (20) puisse être sélectionnée ou ajustée en tant que passage d'air en faisant tourner la sténose (18).

2. L'appareil de thérapie respiratoire selon la revendication 1, dans lequel la sténose (18) possède une paroi (21) par laquelle l'accès au corps creux (3) dans la direction de son axe de symétrie (3') est fermé, et dans lequel le contour extérieur de la sténose (18) est adapté au contour intérieur du corps creux (3).

3. L'appareil de thérapie respiratoire selon l'une des revendications 1 ou 2, dans lequel les ouvertures de passage (20) peuvent être fermées au moyen d'un bouchon (23).

4. L'appareil de thérapie respiratoire selon l'une des revendications précédentes, dans lequel la languette (15) repose sur la surface du corps creux (13) ou se soulève d'un côté de celle-ci, ou dans lequel la languette (15) permet à l'air d'entrer et/ou de sortir par l'ouverture (14) de manière oscillante.

5. L'appareil de thérapie respiratoire selon l'une des revendications précédentes, dans lequel un premier et un second corps oscillant (11, 12) sont disposés dans une direction respiratoire et/ou dans la direction respiratoire opposée.

6. L'appareil de thérapie respiratoire selon l'une des revendications précédentes, dans lequel le corps creux (13) est conçu comme un profilé de boîte qui enferme une cavité de façon cubique, dans lequel un capteur (24) et/ou un microphone (25) est connecté à au moins un des corps oscillants (11, 12), dans lequel des signaux acoustiques ou électriques sont transmis à un appareil externe (26), des signaux acoustiques ou électriques sont transmis à un appareil externe (26), les corps oscillants (11, 12) servant de corps sonores acoustiquement perceptibles pour vérifier l'utilisation correcte ou souhaitée de l'appareil de thérapie respiratoire (1), et les ouvertures étant prévues sur une face frontale du boîtier, parallèlement aux valves.

7. L'appareil de thérapie respiratoire selon l'une des revendications précédentes, dans lequel la languette (15) est faite d'un matériau flexible, de préférence en métal, en plastique dur ou en tissu fait de ces matériaux.
